**Europäisches Patentamt**

(19) **European Patent Office**

**Office européen des brevets**

(11) Publication number: **0 033 628**
**A2**

(12) **EUROPEAN PATENT APPLICATION**

(21) Application number: **81300345.6**

(22) Date of filing: **27.01.81**

(51) Int. Cl.³: **A 61 K 6/04**
**C 22 C 5/06, C 22 C 30/04**

(30) Priority: **31.01.80 AR 279815**

(43) Date of publication of application:
**12.08.81 Bulletin 81/32**

(84) Designated Contracting States:
**AT BE CH DE FR GB IT LI LU NL SE**

(71) Applicant: **MACRODENT S.A.**
**Heredia 665**
**1427 Buenos Aires(AR)**

(72) Inventor: **Pokorni, Tomas Peter**
**Balcarce 2481 1640 Martinez**
**Buenos Aires(AR)**

(72) Inventor: **Balzaretti, Daniel Eduardo**
**Agustin Alvarez 1638 Vincente Lopez**
**Buenos Aires(AR)**

(74) Representative: **Geldard, David Guthrie et al,**
**URQUHART-DYKES AND LORD 11th Floor, Tower**
**House Merrion Way**
**Leeds, LS2 8PB West Yorkshire(GB)**

(54) **A product to be used in dental amalgams.**

(57) A new amalgamable powder is disclosed containing, in the preferred embodiment, by weight, a mixture of between 24% and 56% silver, 20% and 48% tin, and 12.5% and 43% copper, in which when the tin/copper relation is between 1/1 and 2.50/1, the silver/tin relation is between 0.75/1 and 1.8/1, and when the tin/copper relation is between 0.75/1 and 1./1, the silver/tin relation is between 0.75/1 and 2.5/1. In the amalgamable powder at least a portion of the particles are of irregular polyhedral shape, obtained by mechanical means starting from a solid state alloy, while the remainder of the particles, if any, are spheroidal shape, obtained by rapid solidification from a liquid state. In addition, at least 80% of the particles of the powder have their largest dimension greater than 4 microns.

COMPLETE DOCUMENT

Croydon Printing Company Ltd.

## A PRODUCT TO BE USED IN DENTAL AMALGAMS

This invention relates to dental alloy powders and to dental amalgams made therefrom.

Conventional silver alloy powders used to prepare dental amalgams contain a minimum of approximately 65% silver and maximums of approximately 6% copper, 29% tin, 2% zinc and 3% mercury. These powders may comprise particles of either irregular or approximately spherical form and the particles may vary in size. The mixing of one of these powders with mercury in a proportion of 45% to 55% forms a dental amalgam which hardens in a few minutes, allows conformation by carving in a period of a few more minutes and almost totally completes its reaction in 24 hours, developing during that time properties which hardly vary later.

The essential part of these "conventional" alloys, which are of limited copper content, is the inter-metallic compound Ag3Sn, which contains approximately 73% silver and 27% tin and is generally referred to as "gamma" phase. When this compound reacts with mercury, two new solid phases are formed, one of silver mercury (Ag2Hg3), named "gamma 1", and the other of tin mercury (Sn7Hg or Sn8Hg), named "gamma 2", all of which constitute the matrix of the amalgam; some remains of particles from the original "gamma" phase which did not react are also to be found within the matrix.

A dental amalgam must satisfy two types of requirements. During preparation, placing "in situ" and shaping by carving, it should have sufficient plasticity and reaction speed to enable the dentist, using appropriate instruments, to place the amalgam in the cavity, with perfect adaption to same, and have the necessary time to condense and carve it correctly. Once the amalgam has completed its reaction, it should not be biologically incompatible and should restore the tooth to its original function, maintaining during a long period of time its shape, colour and appearance, for which it must possess sufficient mechanical properties and adequate corrosion resistance.

The differences between various conventional alloys are mainly due to the shape and size of their particles, rather than due to their chemical composition. Conventional alloys differ from one another mostly in their handling characteristics. The use of only spherical particles in such alloys bestows greater plasticity to the amalgams, making it necessary to apply less pressure, when condensing, in order to avoid dislodging the plastic mass from the cavity. This generally provokes an incorrect adaption of the amalgam to the tooth. Such is not the case when only irregular particles are used in the alloys. The latter condition not only allows, but requires, more pressure to be used when condensing.

On equal terms of composition and working conditions, spherical particle alloys require less mercury than those of irregular particles. Under equal conditions, spherical particle amalgams contract during reaction, whereas those of irregular particles expand.

The main causes of failures in amalgam dental restorations are marginal fractures, excessive expansion or contraction and pigmentation or corrosion of the restoration. Investigations and tests made during recent years have shown that resistance to "creep" (plastic deformation under moderate compressive strength) is the most important mechanical property in amalgams. Comparative laboratory tests and clinical evaluations made with different amalgams have established a highly significant correlation between increased (or high) resistance to "creep" and low incidence of marginal fractures in dental amalgam restorations.

Following "creep", in order of importance of amalgam mechanical properties, are compressive strength (the final value or the value reached at 60 minutes after preparation of the amalgam) and dimensional stability.

Limitations in the composition of conventional amalgam alloys were based on the theory that, if by increasing the copper content this improved the resistance to "creep" and compression, such an increase of copper content (or lessening of the silver/copper proportion) could affect the corrosion resistance of amalgams. Intuitive or casual tests carried out by some dentists led them to add an additional proportion of copper to their amalgams, resulting in some cases, in appreciable improvements in the clinical behavior of same.

By mixing conventional alloy powder with a certain proportion of particles of silver/copper eutectic, important prior art improvements were obtained in "creep" and corrosion resistance of dental amalgams, thereby resulting in a clinically superior product. These amalgams are known as "dispersed phase" or "reinforced by dispersion" amalgams. The average composition of the mixture of the two powders in these alloys contains a silver content similar to that of conventional alloys, but has a lower proportion of tin to copper.

Newer prior art experiences and investigations have resulted in an amalgamable powder, the amalgams of which have equal or superior properties to those of the "dispersed phase" amalgams. The newer prior art alloys differ from the dispersed phase ones in that the composition of each of its particles is essentially equal but contains a lower silver/ copper relation (or higher copper content) than that found in the conventional alloys, whereas the proportion of tin remains the same as in the conventional alloys. The particles in the newer alloys tend to be spherical, obtained by means of atomization.

According to one hypothesis, amalgams made with such newer prior art alloys owed their "unexpected" good properties to a difference in concentration of silver and copper on the particle surfaces in relation to the cores.

Due to the totally spherical and very reduced size (less than 44 microns) of the particles, handling characteristics present difficulties in condensing, typical of this type of powder, which, furthermore, cannot be easily mixed with mercury manually or in low energy amalgamators. Also, during reaction these amalgams tend to contract.

Investigations made on amalgams obtained with various of these alloys of "high copper content" revealed that in none of the amalgams with a notably superior resistance to "creep" and corrosion was "gamma 2" phase detected, whereas in all of them, in varying proportions, new phases of copper/tin (Cu6Sn5 or Cu3Sn) showed.

Considering that resistances to compressive strength (expressed in MPa; 1 MPa=10 kg/cm$^2$) of the different phases are approximately as follows: "gamma" : 500 MPa, "gamma 1" : 180 MPa, "gamma 2" : 70 MPa, Cu6Sn5 : 600 MPa, it is easily understandable why mechanical properties improve. Inasmuch as the intermetallic compound Cu6Sn5 is more resistant to corrosion than the "gamma 2" phase, this also explains the improved behavior of this aspect in the alloys.

The object of the present invention is a mixture of amalgamable powders not necessarily obtained, totally or partially, by atomization or other method of abrupt solidification, with a smaller silver/copper relation than usually found in conventional alloys, and from which powders amalgams are obtained having not only better mechanical properties, especially regarding "creep" resistance, but also having greater dimensional stability, high resistance to corrosion and handling characteristics which are easily controllable without requiring the use of certain energy amalgamators.

The improvements and advantages achieved with the amalgamable powder and amalgam of the present invention are due to (1) a well balanced utilization of particles or fractions thereof of a number of different chemical compositions; (2) an adequate proportion of particles of different morphology, of which, among others the polyhedrical (irregular) and spherical types are characteristic; (3) an adequate distribution of different sized particles in a proportional grain wherein the largest dimension of at least 80% of the particles is above 4 microns, and (4) each particle can be, in different grades, heterogenous in its chemical composition as well as covered by a film of different chemical composition and controlled thickness. The amalgamable powder contains as principal constituent elements silver, tin and copper and the average composition of the same are within the following range of weight relations between the different main elements: with a tin/ copper relation of between 1/1 and 2.50/1, the silver/tin relation may vary between 0.75/1 and 1.80/1; with a tin/copper relation of between 0.75/1 and 1/1, the silver/tin relation can vary between 0.75/1 and 2.50/1.

Expressing these proportions in weight percentages of each element, the components are limited between the following minimum and maximum contents: silver between 24% and 56%, tin between 20% and 48% and copper between 12.5% and 43%. The product being a mixture of different powders, some or all of its particles, considered individually, may vary from the foregoing limits provided that the average composition, as a whole, remains within such limits. Preferably, however, the weight percentages of the elements chosen should be as follows: silver between 35% and 47%, tin between 30% and 37%, and copper between 20% and 28%, with the weight relationship between the copper content and the tin content being greater than 0.60/1 but no greater than 0.95/1. The amalgamable powder may also contain as much as, by weight, 2% of zinc, 5% of mercury and up to a 3% total of one, another or any combination of gold, platinum and palladium, distributed in some or all of its particles.

The dental amalgam of the present invention is prepared by mixing the described product, in powder or disintegrable pellet form, with mercury in a powder/mercury ratio of between 1.67/1 and 1/1, which is equivalent to a mercury content of between 37.5% and 50% of the total weight. Preferably, the powder/ mercury proportion is between 1.5/1 and 1.1/1, which is equivalent to a mercury content of between 40% and 48% of total weight.

At least a portion of the particles in the amalgamable powder of the present invention should be of irregular polyhedral shape while the remainder, if any, should be spheroidal in shape. The spheroidal particles may comprise from 0 to 75% of the total weight of the particles in the mixture. Preferably, more than 95%, by weight, of the particles in the mixture have their largest dimension less than 140 microns and more than 30%, by weight, of the particles in the mixture have their largest dimension greater than 44 microns.

The different types of powders, spheroidal and irregular, which make up the mixture may be obtained by different means. The spheroidal type, in which the majority of particles are spheres, spheroids, ovoid, lenticular, agglomerated spheroids, cylinders, etc., characteristically, in all its forms, lacks edges or definite angles and is obtained by means of a rapid solidification of a pulverized liquid alloy.

The irregular shaped particles are made up of irregular grains having multiple flat facets. They tend to have polyhedric forms, with or without sharp edges, and are obtained by mechanical means or working, starting from a solid state alloy.

Once the different individual powders of the alloy have been prepared, one or all of them may be submitted to separate additional treatments and/or processes which tend to modify the homogeneous or heterogenous conditions of the composition in different zones of each particle (e.g., its surface covering).

Preferably, at least some of the particles in the mixture are chemically heterogeneous, the silver composition of such particles showing a variable concentration gradient from the core to the surface of such particles, and the exterior of such particles showing a higher content of silver or other precious metals or both. In addition, it is preferable that at least some of the particles in the mixture are superficially covered by silver, gold, palladium or platinum, or by amalgamable alloys thereof, which participate significantly in the amalgamization, whether by inter-alloying or by means of another alloying element.

The amalgamable powder of the present invention may be agglomerated into disintegrable pellets or tablets for convenient use, or may be used in its powder form.

The mixing together of powders having different chemical compositions, morphology and grains gives rise to the possibility of achieving, simultaneously, the following advantages and improvements in amalgams: (a) good wetting properties of the resulting powder with mercury, due to the presence of some particles with greater affinity to the mercury because of, for example, the composition or the surface covering selected, all of which make possible easier manual or low energy mechanical trituration using lower proportions of mercury; (b) adequate plasticity of the amalgam during handling, achieved by selecting a suitable mixture of different shaped and sized particles, which allows condensing the amalgam with little effort but with sufficient pressure to make the amalgam conform well to the cavity form; (c) optimal working time, obtained through an adequately well-balanced combination of size, composition and surface covering of the particles; (d) rapid increase of resistance to compression in newly prepared amalgams, achieved by the presence of a sufficient proportion of fine grains of appropriate composition, which protects the amalgams from fractures due to premature usage; (e) excellent final mechanical properties, especially with regard to compressive strength and "creep", achieved by the virtual elimination of the weak "gamma 2" phase, by a reduction in the amount of "gamma 1" phase present, the gamma 1" phase being reinforced by copper/tin intermetallic compounds, and by a notable reduction of the porosity, made possible, respectively, by an adequate tin/ copper relation, by a smaller proportion of mercury necessary for amalgamation and by an adequate composition in the grain and morphology of the powder; (f) excellent dimensional stability achieved by the equilibrium of the opposed tendencies between the larger and irregular particles to the expansion of the amalgam and those of the smaller and spherical ones to the contraction; and (g) high resistance to corrosion by the virtual absence of the corrodable "gamma 2" phase.

With the object of better illustrating the characteristics of amalgamable powder of this invention and the improvements in the properties of the amalgams prepared with it, a specific example is described below with the express understanding that the same is included herein only to better illustrate the invention and is in no way intended to limit the scope of the invention as presented in the general description and appended claims.

## E X A M P L E

A mixture in equal parts of two amalgamable powders is employed in this example. One of the powders is prepared by the atomization, in an argon gas atmosphere, of a molten alloy containing silver, tin and copper in the following relations: silver/tin in a 1.60/1 relation and tin/copper in a 1.18/1 relation, from which a composition results, expressed in weight percentages, of 24.5% copper, 29% tin and 46.5% silver, approximately. The particles in this powder are of a predominantly spherical form and sizes vary from 74 microns to less than 1 micron with more than 70% of the particles, by weight, having sizes inferior to 37 microns. The other powder is prepared by mechanical means, starting from ingots or bars composed of silver, tin and copper in the following relations: silver/tin in a 1.32/1 relation and tin/copper in a 1.40/1 relation, which results in an alloy composed of approximately 23.5% copper, 33% tin and 43.5% silver, by weight, from which are separated out those particles retained in an ASTM NO. 140 mesh sieve and those that pass through an ASTM NO. 325 mesh sieve, the remaining particles being approximately 45 microns to 120 microns in size.

Amalgams made with approximately 47% mercury and 26.5% of each of the powders described above, in total weight doses of approximately 1.13 grams, were placed in a plastic capsule containing a steel pestle weighing 1.10 grams and triturated for 10 seconds by an apparatus which vibrated at a rate of approximately 3000 revolutions per minute.

These amalgams were condensed into appropriate specimens, and tested according to methods specified in the American Dental Association's Specification No. 1. A compressive strength of 147 MPa was measured at 60 minutes, 451 MPa at 24 hours and 521 MPa at 7 days; "creep" deformation at 7 days was 0.04%; and the dimensional change at 24 hours was 0.01%.

It is necessary to point out that the specification referred to requires the following values: minimum compressive strength at 60 minutes: 80 MPa; maximum "creep" deformation: 5.00%; maximum dimensional change: 0.20%.

Corrosion tests in artificial saliva of the specimen amalgams, compared with identical tests of "dispersed phase" and conventional amalgams, showed that the specimen amalgam and the dispersed phase amalgam had considerably lower dissolution timing than the conventional type. The stationary current generated in the test specimen and in "dispersed phase" type amalgams, from an initial current until a limiting current, involves an increase of less than 1 order of magnitude, whereas in conventional amalgams an increase of approximately 4 orders of magnitude ($2x10^{-4}$ to 2 $mA/cm^2$) is encountered. The test specimens were submitted to metallographic observations, chemical and X-Ray tests and in no case was the presence of "gamma 2" phase detected.

Very similar results in regard to mechanical properties, resistance to corrosion and absence of "gamma 2" phase were obtained with the same mixture of powders and mercury, but with the following preparation methods: (a) by manual mixing with glass mortar and pestle, triturating during 40 seconds; and (b) by previously preparing a number of pellets of approximately 0.30 grams each in weight, each pellet containing equal parts of each powder, and placing two of these pellets with 0.53 grams of mercury into the same capsule as used in the "Example" described earlier, with the same pestle, and vibrating the capsule in the same apparatus as used in the "Example" for a 16 second period.

In each of the three tests, handling characteristics were found to be adequate, allowing reasonable working time and adapting excellently to the cavity into which the amalgam was inserted and condensed manually.

Various tests were made in which the following parameters were varied within the limits outlined in the foregoing description of the invention: (a) changing the composition of one or both of the powders involved, (b) forming mixtures of more than two powders (with the average compositions of the mixtures meeting the criteria set forth earlier in the description of the invention), (c) forming mixtures of two or more powders having identical compositions but different morphologies of their particles, (d) forming powders composed of particles of the same form (irregular) and composition, but of different sizes, (e) forming mixtures of powders having different degrees of heterogeneity of their particles, (f) preparing amalgams having mercury proportions varying between 37.5% and 50%, and g) varying the times, vibration speeds and means of trituration of the amalgam.

Although some results were observed during the foregoing tests which were not as good as others, in no case was "Gamma 2" phase observed and in all cases resistance to corrosion and creep, as well as all other mechanical properties, was notably superior to the results obtained from conventional silver alloy amalgamable powders.

0033628

- 10 -

CLAIMS:

1. A product to be used in dental amalgams and containing, by weight, a mixture of between 24% and 56% silver, 20% and 48% tin, and 12.5% and 43% copper, characterized in that:

(a)  the weight relations between such components in the final mixture are as follows:

| When the Tin/Copper Relation is Between | The Silver/Tin Relation May Vary Between |
| --- | --- |
| 1/1 and 2.50/1 | 0.75/1 and 1.8/1 |
| 0.75/1 and 1/1 | 0.75/1 and 2.5/1 |

(b)  at least a portion of the particles of the product are of irregular polyhedral shape, obtained by mechanical means starting from a solid state alloy and the remainder, if any, of the particles of the product are spheroidal in shape, obtained by rapid solidification from a liquid state, and

(c)  at least 80%, by weight, of the particles in the mixture have their largest dimension greater than 4 microns.

2. A product to be used in dental amalgams, according to claim 1 containing, by weight, between 35% and 47% silver, 30% and 37% tin, and 20% and 28% copper, the weight relationship between the copper content and the tin content being greater than 0.60/1 but no greater than 0.95/1.

3. A product to be used in dental amalgams, according to claim 1 or claim 2, further including one or more of the following elements in weight amounts of up to 2% of zinc, up to 5% of mercury, and up to 3% total of one, another, or any combination of gold, platinum and palladium.

4. A product to be used in dental amalgams, according to any one of the preceding claims characterized by the fact that spherical particles comprise 0 to 75% of the total weight of the particles in the mixture.

5. A product to be used in dental amalgams, according to any one of the preceding claims characterized by the fact that more than 95%, by weight, of the particles in the mixture have their largest dimension less than 140 microns and more than 30%, by weight, of the particles in the mixture have their largest dimension larger than 44 microns.

6. A product to be used in dental amalgams, according to any one of the preceding claims, characterized by the fact that at least some of the particles in the mixture are chemically heterogeneous, the silver composition of such particles showing a variable concentration gradient from the core to the surface of the particles, and the exterior of such particles showing a higher content of silver or other precious metals or both.

7. A product to be used in dental amalgams, according to any one of the preceding claims, characterized by the fact that at least some of the particles in the mixture are superficially covered by silver, gold, palladium or platinum or by amalgamable alloys thereof, wherein these metals have an important individual participation, be it inter-alloyed or by means of another alloying element.

8. Disintegrable pellets or tables formed by the agglomeration of the product claimed in any one of the preceding claims.

9. A dental amalgam for use in dental restorations and obtained by mixing the product claimed in any one of claims 1 to 7 with mercury, the amalgam containing between 37.5% and 50% of mercury, by weight.